# EUROPÄISCHE PATENTANMELDUNG

(11) **EP 1 010 408 A1**
(43) Veröffentlichungstag der Anmeldung: **21.06.2000**
(21) Anmeldenummer: 99113401.6
(22) Anmeldetag: 12.07.1999
(51) Int. Cl.: A61F 5/01

(54) **Knieorthese**

(30) Priorität: 17.12.1998 DE 29822445 U
(71) Anmelder: IPOS GmbH & CO. KG., D-21337 Lüneburg (DE)
(72) Erfinder: Prahl, Jan, 21379 Rullstorf (DE)
(74) Vertreter: Richter, Werdermann & Gerbaulet

(57) **Zusammenfassung**

Um eine Knieorthese (100) mit wenigstens einem Gelenk (10), welches zwei an einem Drehpunkt (17) miteinander schwenkbar verbundene Schienen (12) aufweist, wobei an wenigstens einer Schiene (12) ein Anschlag (14) vorgesehen ist, welcher eine maximale Streckbewegung des Gelenkes (10) begrenzt, zu schaffen, welche den Tragekomfort sowie die Akzeptanz beim Patienten erhöht, wird vorgeschlagen, dass wenigstens eine der das Gelenk (10) bildenden Schienen (12) benachbart zum Anschlag (14) wenigstens einen Schlitz (16) mit einer offenen Seite (18) und gegenüberliegenden Schlitzwänden (20) aufweist, welcher derart angeordnet ist, dass ein Anschlagen der Schienen (12) aneinander bei einer maximalen, vorbestimmten Streckposition die Schlitzwände (20) unter elastischer Verformung eines Werkstoffes der Schiene (12) aufeinander zu bewegt.

## Beschreibung

Die Erfindung betrifft eine Knieorthese mit wenigstens einem Gelenk, welches zwei an einem Drehpunkt miteinander schwenkbar verbundene Schienen aufweist, wobei an wenigstens einer Schiene ein Anschlag vorgesehen ist, welcher eine maximale Streckbewegung des Gelenkes begrenzt, gemäß dem Oberbegriff des Anspruchs 1.

Knieorthesen dienen zum Stützen eines Kniegelenkes eines Patienten, beispielsweise zum Schutz vor Verletzungen oder eine Behandlung eines Knieleidens bzw. einer Knochen- oder Bänderverletzung im Kniebereich postoperativ begleitend. Herkömmliche Knieorthesen haben den Nachteil, dass beim Erreichen eines eine maximale Streckbewegung begrenzenden Anschlages im Gelenk ein hörbares und vom Träger als unangenehm empfundenes Geräusch entsteht.

Es ist Aufgabe der vorliegenden Erfindung, eine verbesserte Knieorthese der obengenannten Art zur Verfügung zu stellen, welche die obengenannten Nachteile beseitigt und einen Tragekomfort sowie eine Akzeptanz beim Patienten erhöht.

Diese Aufgabe wird durch eine Knieorthese der o.g. Art mit den in Anspruch 1 gekennzeichneten Merkmalen gelöst.

Dazu ist es erfindungsgemäß vorgesehen, dass wenigstens eine der das Gelenk bildenden Schienen benachbart zum Anschlag wenigstens einen Schlitz mit einer offenen Seite und gegenüberliegenden Schlitzwänden aufweist, welcher derart angeordnet ist, dass ein Anschlagen der Schienen aneinander bei einer maximalen, vorbestimmten Streckposition die Schlitzwände unter elastischer Verformung eines Werkstoffes der Schiene aufeinander zu bewegt.

Dies hat den Vorteil, dass eine Geräuschbildung beim Erreichen des Anschlages wesentlich reduziert ist, wodurch sich ein Tragekomfort der Knieorthese und damit eine Akzeptanz der Knieorthese durch einen Patienten erheblich verbessert.

Vorzugsweise Weitergestaltungen der Vorrichtung sind in den Ansprüchen 2 und 3 beschrieben.

Eine reversibles Zusammendrücken des Schlitzes ausschließlich innerhalb eines Hookeschen Bereiches eines Dehnungsdiagrammes des Werkstoffes der Schiene erzielt man dadurch, dass der Schlitz in seiner Länge und seiner Breite derart dimensioniert ist, dass die Schlitzwände aneinander Anschlagend ein Zusammendrücken des Schlitzes stoppen, bevor eine plastische Verformung des Werkstoffes der Schiene erfolgt.

Der Werkstoff der Schienen ist beispielsweise Titan oder Stahl.

Nachstehend wird die Erfindung anhand der beigefügten Zeichnungen näher erläutert. Diese zeigen in
- Fig. 1: ein bevorzugte Ausführungsform einer erfindungsgemäßen Knieorthese in perspektivischer Darstellung,
- Fig. 2: eine erste bevorzugte Ausführungsform von Gelenkschiene für eine erfindungsgemäße Knieorthese in Aufsicht,
- Fig. 3: eine zweite bevorzugte Ausführungsform von Gelenkschiene für eine erfindungsgemäße Knieorthese in Aufsicht,
- Fig. 4: eine dritte bevorzugte Ausführungsform von Gelenkschiene für eine erfindungsgemäße Knieorthese in Aufsicht,
- Fig. 5: eine bevorzugte Ausführungsform einer erfindungsgemäß ausgebildeten Schiene für eine erfindungsgemäße Knieorthese in Seitenansicht und
- Fig. 6: eine vergrößerte Detailansicht eines Anschlages der Schiene von Fig. 5 in Seitenansicht.

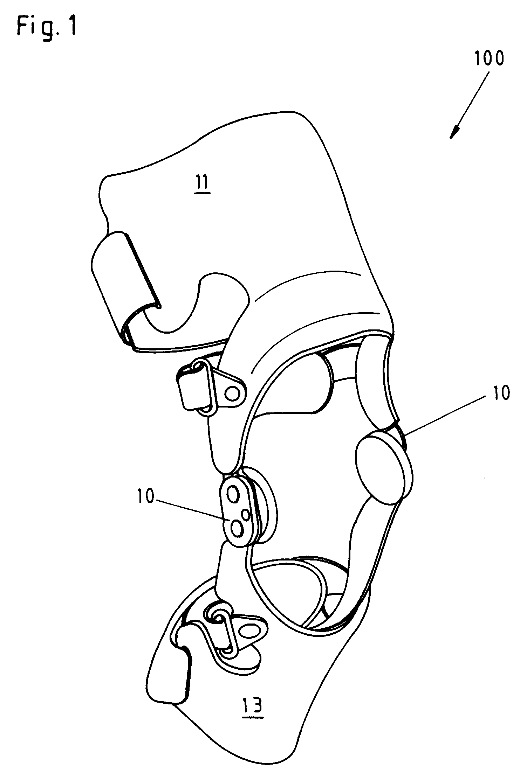

Die in Fig. 1 dargestellte bevorzugte Ausführungsform einer erfindungsgemäßen Knieorthese 100 umfasst ein Oberschenkelteil 11, ein Unterschenkelteil 13 sowie zwei Gelenke 10, welche jeweils von zwei in Fig. 1 nicht sichtbaren Schienen ausgebildet sind und das Oberschenkelteil 11 mit dem Unterschenkelteil 13 schwenkbar verbinden.

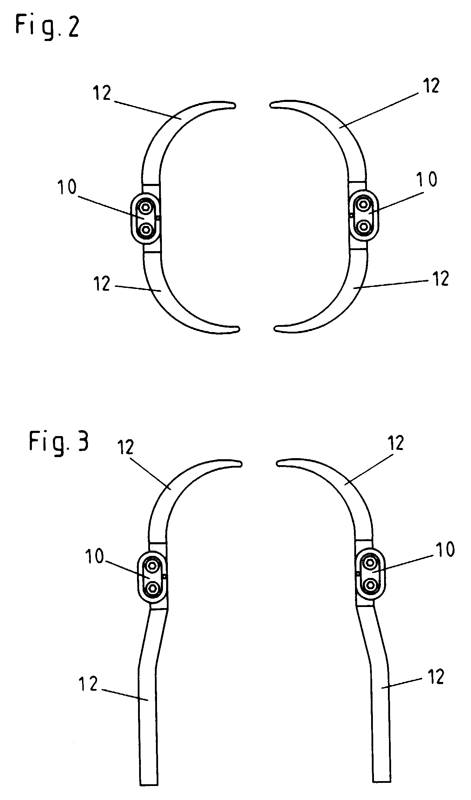

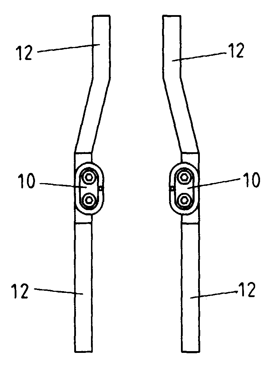

Die Fig. 2 bis 4 illustrieren verschiedene Ausführungsformen für gelenkbildende Schienen 12 für unterschiedliche Knieorthesen. So ist die zu den Schienen 12 gemäß Fig. 2 gehörende Knieorthese beispielsweise bei Ruptur eines vorderen Kreuzbandes, ein- oder mehrachsigen Instabilitäten des Kniegelenkes, bei Verletzung des Innen- oder Außenbandapparates bzw. bei frühfunktioneller Nachbehandlung indiziert. Die zu den Schienen 12 gemäß Fig. 3 gehörende Knieorthese ist beispielsweise bei Ruptur des hinteren Kreuzbandes bzw. bei Verletzung des Innen- oder Außenbandapparates indiziert. Die zu den Schienen 12 gemäß Fig. 4 gehörende Knieorthese ist beispielsweise bei Gonarthrose, Instabilität nach Kniegelenkersatz (TEP) bzw. genu recurvatum indiziert.

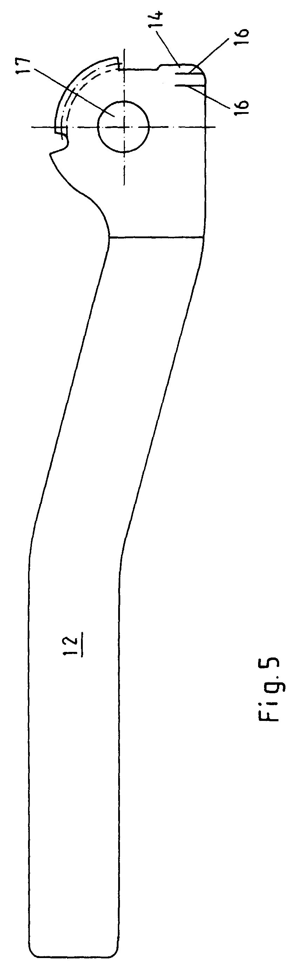

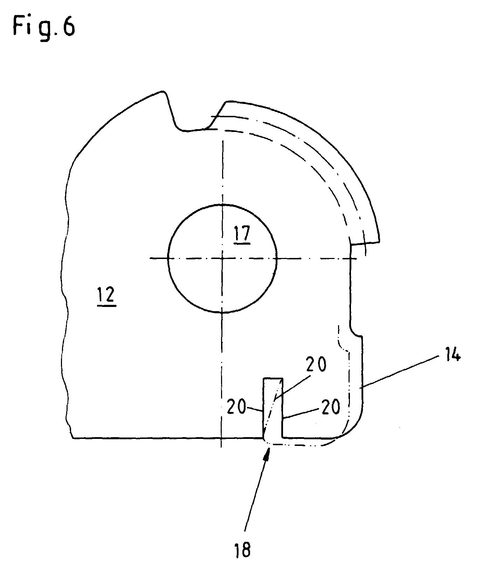

Wie aus Fig. 5 und 6 ersichtlich sind bei wenigstens einer Schiene mit einem Drehpunkt 17 benachbart zu einem Anschlag 14 zwei Schlitze 16 mit einer offenen Seite 18 und gegenüberliegenden Schlitzwänden 20 ausgebildet, wobei die Schlitze 16 derart angeordnet sind, dass ein Anschlagen der Schienen 12 aneinander bei einer maximalen, vorbestimmten Streckposition die Schlitzwände 20 unter elastischer Verformung eines Werkstoffes der Schiene 12 aufeinander zu bewegt. Dies ist in Fig. 6 mit gestrichelten Linien angedeutet. Die Schlitze 16 werden beispielsweise mit einem Laser im Anschlagbereich eingebracht und drücken sich beim Anschlag zusammen, was eine Geräuschdämpfung bewirkt. Erste Versuche haben eine Geräuschdämpfung von ca. 50% gezeigt.

Die erfindungsgemäß vorgesehenen Dämpfungsschlitze 16 sind schmaler ausgebildet als ein Hookescher Bereich im Dehnungsdiagramm. Beim Zusammendrücken des Schlitzes 16 wird dabei eine gerade Strecke des Dehnungsdiagramms, in der die elastische Dehnung proportional zur Spannung ist, nicht überschritten. Die Schlitzwände bzw. Flanken 20 des Schlitzes 16 kommen vorher zur Anlage und bewirken eine Dauernutzung ohne Verschleiß.

## Patentansprüche

1. Knieorthese (100) mit wenigstens einem Gelenk (10), welches zwei an einem Drehpunkt (17) miteinander schwenkbar verbundene Schienen (12) aufweist, wobei an wenigstens einer Schiene (12) ein Anschlag (14) vorgesehen ist, welcher eine maximale Streckbewegung des Gelenkes (10) begrenzt,
dadurch gekennzeichnet,
dass wenigstens eine der das Gelenk (10) bildenden Schienen (12) benachbart zum Anschlag (14) wenigstens einen Schlitz (16) mit einer offenen Seite (18) und gegenüberliegenden Schlitzwänden (20) aufweist, welcher derart angeordnet ist, dass ein Anschlagen der Schienen (12) aneinander bei einer maximalen, vorbestimmten Streckposition die Schlitzwände (20) unter elastischer Verformung eines Werkstoffes der Schiene (12) aufeinander zu bewegt.

2. Knieorthese (100) nach Anspruch 1,
dadurch gekennzeichnet,
dass der Schlitz (16) in seiner Länge und seiner Breite derart dimensioniert ist, dass die Schlitzwände (20) aneinander Anschlagend ein Zusammendrücken des Schlitzes (16) stoppen, bevor eine plastische Verformung des Werkstoffes der Schiene (12) erfolgt.

3. Knieorthese (100) nach Anspruch 1 oder 2,
dadurch gekennzeichnet,
dass der Werkstoff der Schienen (12) Titan oder Stahl ist.
